# EUROPEAN PATENT APPLICATION

(11) **EP 2 636 401 A1**
(43) Date of publication of application: **11.09.2013**
(21) Application number: 13158319.7
(22) Date of filing: 08.03.2013
(51) Int. Cl.: A61K 8/04, A61K 8/06, A61Q 17/04, A61Q 19/00, A61Q 19/04

(54) **Aerosol container comprising a dermatological composition and a foaming agent**

(30) Priority: 09.03.2012 NL 2008443
(71) Applicant: DREUMEX B.V., 5347 JK Oss (NL); JAO beheer BV, 6097 CR Heel (NL); Beckman Lapre Beheer BV, 8621 CV Heeg (NL)
(72) Inventor: Hilgers, Marcus Joseph Johannes, 6074 HX Melick (NL); van Krieken, Thomas, 5025 DG Tilburg (NL); Derks-Fens, Irene, 4852 Hombourg (BE); Gelinne, Marleen, 3001 Heverlee (BE)
(74) Representative: EP&C

(57) **Abstract**

Bag-on-valve aerosol container defining a chamber, wherein a pressurized gas is contained, surrounding a flexible bag, and wherein a closable outlet is in fluid communication with said flexible bag, said flexible bag comprising a leave-on dermatological composition comprising:
- an oil-in-water emulsion, comprising one or more oils, the emulsion having an oil phase to water phase ratio of between 1:1 and 1:25;
- one or more oil-in-water emulsifiers; and
- one or more active dermatological ingredients;
wherein said leave-on dermatological composition has a viscosity at room temperature of between 1 and 20,000 mPa.s and wherein said product compartment further comprises one or more physical foaming agents having a standard boiling point of -50 to 70 °C.

## Description

### Technical Field

The present invention relates to aerosol containers comprising a dermatological composition and a foaming agent.

More in particular, the present invention relates to aerosol containers having a flexible inner product compartment comprising a dermatological composition, which comprises an oil-in-water emulsion, one or more oil-in-water emulsifiers and one or more active ingredients.

### Background Art

It is known in the art to use aerosol containers to deliver dermatological compositions. Dermatological compositions can be divided into compositions that are to be removed from the skin after application and compositions that are intended to be left on the skin, i.e. so-called stay-on or leave-on dermatological compositions.

It also known to deliver dermatological compositions in the form of a foam, e.g. a foaming sunscreen composition.

Foaming leave-on dermatological compositions delivered by state of the art aerosol containers come in many different forms and textures. The quality of such foams leaves something to be desired, i.e. the delivered foams tend to be too dry, coarsely-bubbled and/or only slightly stable, leading to the composition simply dripping or flowing from the skin after application. A suitable foam should be easy to spread, have a certain density associated with it and stand for at least the time a person needs to spread the foam over the skin. Further, a suitable foam should adhere to the skin without being too sticky. A foam that fulfils these criteria is, in the context of the present application, denoted a rich foam.

EP1508326 seeks to provide a solution for the above disadvantages of current foaming cosmetic and dermatological compositions. It is related to the use of UV filter substances for optimizing the quality of self-foaming, foam-like, after-foaming or foamable cosmetic and dermatological preparations. The described compositions can be emulsions. Said preparations are preferably removed from two-chamber aerosol containers, in which there is one chamber with a filling of the liquid or slurry-like preparations that may comprise a secondary propellant, under the pressure of a primary propellant located in a second chamber, enclosing the first chamber. Bican^{®} aerosol containers in which the product is enclosed in a flexible bag are mentioned as an example. It is said that when secondary propellants are used in combination with said UV-filters, particularly propellants soluble in the oil phase, e.g. customary propane/butane mixtures, the preparations described are not simply sprayed as aerosol droplets, but develop into finely-bubbled, rich foams as soon as preparations containing such propellants experience decompression.

Although EP1508326 in certain cases indeed delivers a rich foam, there are some disadvantages associated with the provided solution. As it turns out, it is not always convenient or needed to have one or more of the particular UV-filters present in dermatological compositions. In addition, in practice it turns out that not every dermatological composition comprising one or more UV-filters and a secondary propellant leads to a suitable foam, i.e. a foam that is rich enough as not to drip from the skin and that allows for easy spreading.

In EP1391192 an alternative solution is proposed. Also in this case a dermatological composition comprising a secondary propellant may be contained in a flexible bag that is surrounded by an aerosol container that is pressurized with a primary propellant. In order to arrive at a suitable foaming performance the patent prescribes that an emulsifier system must be employed that consists of three different emulsifiers. It is evident that the use of three different emulsifiers makes the composition complex and potentially unsuitable for several uses.

### Summary of the Invention

It is an object of the present invention to provide a barrier pack aerosol container that is able to deliver a leave-on dermatological composition that is expelled as a rich foam, without the necessity to use one or more UV-filters as a foaming agent or the need to employ three different emulsifiers.

These objects are achieved by a bag-on-valve aerosol container defining a chamber, wherein a pressurized gas is contained, surrounding a flexible bag, and wherein a closable outlet is in fluid communication with said flexible bag, said flexible bag comprising a leave-on dermatological composition comprising:
- an oil-in-water emulsion, comprising one or more oils, the emulsion having an oil phase to water phase ratio of between 1:1 and 1:25;
- one or more oil-in-water emulsifiers; and
- one or more active dermatological ingredients;
wherein said leave-on dermatological composition has a viscosity at room temperature of between 1 and 20,000 mPa.s and wherein said product compartment further comprises one or more physical foaming agents having a standard boiling point of -50 to 70 °C.

It has been surprisingly found that when the viscosities of the compositions are inside the viscosity range claimed, the compositions provide a satisfactory foaming behaviour, whereas compositions with a different viscosity fail to provide such foaming perfermance. The presence of UV filter compounds or the presence of three or more emulsifiers does not have an effect provided that the viscosity of the composition is within the range according to the present invention.

It is known from US 6620855 to provide a container containing a pressurised gas and an emulsion with a fluidity comparable with that of water, such as with a viscosity below 20 mPa.s, in a transparent aerosol receptacle. Thereto, the receptacle is loaded with the emulsion and subsequently, pressurised with the gas, such as isobutane. Neither the receptacle nor the emulsion fulfils the requirements of the present invention.

Preferably, the one or more physical foaming agents have a standard boiling point of - 50 to 40 °C, and are suitably selected from propane, butane, isobutane, pentane, isopentane, dimethyl ether, diethyl ether and mixtures thereof. The leave-on dermatological compositions contained in the aerosol containers preferably have a viscosity at room temperature of between 50 and 20,000 mPa.s, more preferable between 100 and 15,000 mPa.s. Advantageously, the leave-on dermatological composition contained in the bag-on-valve aerosol containers is a skin care formulation, examples of which are a body lotion, a sunscreen formulation, an after sun formulation, a tanning lotion and a baby care composition such as a nappy rash cream.

The invention also provides a method for filling the bag-on-valve aerosol container, comprising the steps of:
(a) pressurizing a can with a pressurizing gas;
(b) bringing a valve assembly comprising a flexible bag, a valve and a disc into the can such that the pressurizing gas surrounds the flexible bag;
(c) attaching the valve assembly with its disc to the can such that the aerosol container is closed; and
(d) filling through the valve one or more physical foaming agents as liquids into the flexible inner bag; and
(e) subsequently adding thereto the leave-on dermatological composition. The dermatological composition to be filled into the container via this method is suitably a leave-on dermatological composition that has a viscosity at room temperature of between 1 and 20,000 mPa.s, and is advantageously a leave-on-dermatological composition as described above.

This method surprisingly has the advantage that the filling speed can be increased significantly, in particular in comparison with the embodiment wherein the foaming agents and the emulsion are combined prior to being added to the flexible bag. It would be disadvantageous to fill the flexible bag with emulsion before adding the foaming agent, since, the liquid foaming agent would potentially not mix sufficiently with the emulsion and be sprayed from the aerosol prior to the spraying of the emulsion, thereby negatively affecting the foaming of the composition. A particular advantage of employing the filling method according to the invention for dermatological compositions as described above, with a viscosity of 1 to 20,000 mPa.s resides in that such compositions readily and spontaneously mix with the physical foaming agents that have been introduced into the flexible inner bag previously. The spontaneous mixing benefits the foaming behaviour when the composition is released from the aerosol container.

### Detailed Description

The present invention provides a bag-on-valve aerosol container comprising a leave-on dermatological composition as defined having a viscosity between 1 and 20,000 mPa.s and one or more physical foaming agents having a standard boiling point of between -50 to 70 °C.

Surprisingly, the inventors found that the combination of one or more physical foaming agents and a leave-on dermatological composition having the desired viscosity leads to a rich foam upon discharging the aerosol container. In the following, the combination of a dermatological composition and one or more physical foaming agents will be referred to as a "formulation".

Leave-on dermatological compositions that are applied as a foam have a clear advantage over their unfoamed counterparts. Most people are familiar with the experience of spilling a substantial part of sunscreen lotion because it runs too easily from the skin. When a typical leave-on dermatological composition such as a body lotion or a sunscreen lotion, is applied to the skin, it is difficult to spread the composition evenly and uniformly over those parts of the skin that one intents to rub in. Usually, the spot where the composition is applied, is overloaded, leading to an oily or greasy feeling on that spot, whereas the adjacent parts of the skin are poorly covered, this effect increasing when moving further away from the application spot. Also well-known is the problem that when spreading a clot of a certain skin care composition, it may well be that some spots are left uncovered. In the case of sunscreen lotion, this may result in sun burning of these spots.

These disadvantages are solved at least to a large extent when leave-on dermatological compositions are applied as a foam.

It is essential that the dermatological compositions have a viscosity at room temperature of between 1 and 20,000 mPa.s. Though it might be possible to foam a dermatological composition having a viscosity below 1 mPa.s, it is currently impossible to prepare oil-in-water emulsions that have a viscosity below 1 mPa.s. When the viscosity is higher than 20,000 mPa.s, no foam develops upon discharging the dermatological composition. For an optimal result the viscosity at room temperature preferably is between 50 and 20,000 mPa.s, more preferably between 100 and 15,000 mPa.s. From US 6620855 it is apparent that in the relevant art the viscosity is a known parameter which the skilled person will know how to determine. For the purpose of this application the viscosity is the one obtained by a Brookfield RV viscometer, wherein the viscosity is measured at room temperature (20°C) at the appropriate rotation speed using the appropriate spindle after 1 minute.

The afore-mentioned one or more physical foaming agents should have a standard boiling point of between -50 to 70 °C, whereby 'standard' indicates the boiling point under standard atmospheric conditions. Preferably, the physical foaming agents have a standard boiling point of between -45 to 40 °C, more preferably between -45 to 30 °C. Examples of physical foaming agents include linear, branched or cyclic alkanes having 3-6 carbon atoms, carbon dioxide, dimethyl ether, hydrofluorocarbons, hydrochlorofluorocarbons, hydrofluoroethers, methyl formate and mixtures thereof. Their derivatives may also be used. Preferably, the one or more physical foaming agents are selected from propane, butane, isobutane, pentane, isopentane, dimethyl ether and mixtures thereof.

The aerosol container of the present invention is a bag-on-valve aerosol container, i.e. an aerosol container in which the product is contained in a flexible bag that is attached to the outlet valve and wherein the propellant is contained separate from the formulation. Said aerosol containers as such are known in the art, e.g. from US3823849 and W02005007516. Bag-on-valve aerosols are an example of so-called barrier pack aerosol containers having a flexible inner product compartment.

Said barrier pack aerosol containers find widespread use, for example as bag-in-can aerosol containers (Sepro^{®}, BICAN^{®}) and the more recent bag-on-valve (also known as BOV) aerosol containers, the main difference between the two being the way the inner compartment is attached. In bag-on-valve aerosol containers, a flexible bag is affixed within the container opening either to the aerosol discharge valve or to the bead of the container opening.

In comparison with classic aerosol containers barrier pack aerosol containers having a flexible inner compartment have the advantage of allowing for 360° dispensing, a quiet and non-chilling discharge, a high degree of emptying, a longer shelf life with less preserving agents, a reduced risk of explosion and/or fire due to the absence of flammable and/or explosive propellants.

As said, the invention is related to bag-on-valve aerosol containers. When compared to the above barrier pack aerosol containers, such as bag-in-can or Sepro^{®} aerosol containers, or even piston driven aerosol containers, bag-on-valve aerosol containers have the benefit of prohibiting bleeding of the pressurized gas or propellant from the container. The nature of the pressurized gas is not critical, i.e. any suitable gas may be employed. Examples of suitable gases include alkanes having 3-6 carbon atoms, cyclobutane, cyclopentane, oxygen, dimethyl ether, helium, air and nitrogen. Preferably the pressurized gas is a readily available gas such as air or nitrogen. The pressurized gas surrounds a flexible bag. This bag preferably is made of a flexible material that is impermeable for both the dermatological composition inside the bag and the surrounding pressurized gas. The flexible material of which the inner product compartment is made is not limited to any material in particular. Metal foils such as aluminium foils or thermoplastic polymers, such as polyethylene or polypropylene, are found to be suitable with the present leave-on dermatological compositions.

The flexible bag preferably is a multilayered or laminated bag comprising at least one metal foil layer, preferably an aluminium foil layer, and a thermoplastic layer. For example, the bag may be a laminate comprising four layers, e.g. a polyethylene layer as the innermost layer being in contact with the dermatological composition, then a polyamide layer, an aluminium layer and a polypropylene layer as the outermost layer being in contact with the surrounding pressurized gas. Such a laminated multilayered structure provides a superior impermeability to the bag, making it particularly suitable for application in cosmetic products.

A closable outlet is in fluid communication with the flexible bag. Typically this outlet comprises a valve and an actuator. When the actuator is activated, e.g. pressed, the product comprised in the flexible inner product compartment is discharged through the valve assembly by the driving force the pressurized gas exerts on the flexible inner product compartment. According to the invention, the flexible bag comprises a leave-on dermatological composition that can be foamed. In this context, a dermatological composition is a composition that has a caring, cleansing or decorative effect on the skin. Dermatological compositions can be subdivided in cosmetic and medicinal dermatological compositions. Examples of dermatological compositions include baby care compositions, body lotions, sunscreen lotions, cleansing milks and related compositions.

The leave-on dermatological compositions of the invention comprise an oil-in-water emulsion, one or more oil-in-water emulsifiers and one or more active dermatological ingredients. In this respect, it is noted that the intended use of a dermatological composition influences the choice of ingredients for that dermatological composition. The nature and the amounts of oils and emulsifiers present in a dermatological composition determines the basic characteristics of the dermatological composition, such as spreading, appearance, skin absorption, skin feel, etc. The addition of dermatological active ingredients and/or vitamins promotes specific categories of skin care such as extra nourishing, hydrating, tanning acceleration, masking, etc.

As said, the present invention comprises an oil-in-water emulsion. A water-in-oil emulsion, when employed in an aerosol container of the present invention, provides a watery, unstable foam. Therefore the present invention utilizes oil-in-water emulsions.

Alternatively, the leave-on dermatological composition utilizes one or more oils instead of an oil-water-emulsion. In this case, the leave-on dermatological composition is water-free and the presence of one or more oil-in-water emulsifiers is not required. Preferably, the one or more oils are lipids as defined below.

The oil phase to water phase ratio of the oil-in-water emulsion should be between 1:1 and 1:25. The oil phase typically comprises oils, waxes, oil-soluble emulsifiers and other oil-soluble ingredients. The water phase typically comprises water and other water-soluble ingredients.

The nature of the oil or oils contained in the oil-in-water emulsion is not particularly limited. In fact, every oil or combination of oils commonly used in dermatological compositions may be used. The oil may be a mineral oil, a lipid, a silicone based oil, or a mixture comprising these oils. Examples of lipids include vegetable oils, esters such as triglycerides, ethers such as dicaprylyl ether, fatty acids or phospholipids. Preferably, the oil is a lipid or a silicone based oil. Suitable lipids include, but are not limited to, C₁₂-C₁₅ alkyl benzoates, capric and caprylic triglycerides, diethylhexyl adipate, dibutyl adipate, diisopropyl adipate, diethylhexyl malate, ethylhexyl palmitate, ethylhexyl stearate, isopropyl myristate, isopropyl palmitate, octyldodecyl neopentanoate, PPG-2 myristyl ether propionate, butylene glycol dicaprylate/dicaprate, dicaprylyl ether, triheptanoin, dicaprylyl carbonate and diisopropyl sebacate.

Suitable silicone based oils include cyclopentasiloxane and cyclohexasiloxane, dimethicone and phenyltrimethicone.

Preferably, the oil is selected from C₁₂-C₁₅ alkyl benzoates, ethylhexyl cocoate, dicaprylyl carbonate, dibutyl adipate, cyclopentasiloxane, dimethicone and mixtures thereof.

It is noted that throughout the present application chemical compounds are designated with the name commonly used in the art. This means that sometimes the IUPAC name is used, but that more often INCI (International Nomenclature of Cosmetic Ingredients) names are used.

The nature of the one or more emulsifiers is not particularly limited. In fact, every emulsifier or combination of emulsifiers commonly used in dermatological compositions can be used. Advantageously, the one or more emulsifiers are selected from salt and esters of fatty acids having 10 to 40 carbon atoms, alkyl phosphates having 12 to 20 carbon atoms and their salts, polyglycerol esters, glucosides, sucrose esters and polyethylene glycol esters (PEG) esters. Preferably, the emulsifiers are one or more selected from sorbitan stearate, glyceryl stearate, PEG-100 stearate, potassium cetyl phosphate, polysorbate 80, bis-PEG/PPG-16/16 PEG/PPG-16/16 dimethicone, acrylates/vinyl isodecanoate crosspolymer, ceteareth-20, acrylates/C10-30 alkyl acrylates cross-polymer and laureth-7.

The leave-on dermatological composition according to the invention comprises one or more active dermatological ingredients. In the context of the present invention active dermatological ingredients are compounds that have a caring effect on the skin, whereby it is to be understood that the expression 'caring' should be interpreted broadly. In this definition, the oils and emulsifiers discussed above may also qualify as an active dermatological ingredient. Examples of active dermatological ingredients include moisturizers, nourishing compounds, vitamins, anti-irritants, holding agents, anti-aging agents, slimming agents, exfoliants and anti-oxidants. Preferably, the active ingredients are one or more selected from the group comprising of biosaccharide gum-1, bisabolol, sodium PCA, urea, allantoin, hydrolyzed wheat protein, lactic acid, lysine, PCA, solanum lycopersicum extract, helianthus annuus seed oil, rosmarinus officinalis extract, prunus armeniaca kernel oil, citrus aurantium dulcis extract, tocopheryl acetate and panthenol.

In addition to the above ingredients, the leave-on dermatological compositions according to the invention may also comprise one or more UV-filters, preferably selected from bis-ethylhexyloxyphenol methoxyphenyl triazine, octocrylene, methylene bis-benzotriazolyl tetramethylbutylphenol, ethylhexyl triazone, butyl methoxydibenzoylmethane, ethylhexyl salicylate, phenylbenzimidazole sulfonic acid and diethylhexyl butamido triazone.

A dermatological composition contained in a bag-on-valve aerosol according to the present invention may further comprise auxiliary ingredients such as co-emulsifiers, pH-adjusting agents, preservatives, perfumes, aroma's and rheological additives such as stabilizers, viscosity adjusting agents and film forming agents.

As indicated above, another aspect of the present invention is related to a method for filling the bag-on-valve aerosol containers of the present invention. According to such a method a can is pressurized with the pressurizing gas. Subsequently, a valve assembly comprising a flexible bag, a valve and a disc is brought into the can such that the pressurizing gas surrounds the flexible bag. Simultaneous to inserting the valve assembly into the can the valve assembly is attached to the can with its disc, such that the aerosol container is closed. Then, the one or more physical foaming agents are filled into the flexible inner bag through the valve. Said foaming agents are filled as liquids. Subsequently the dermatological composition is added thereto. Methods and apparatus for pressurizing the can, for bringing the valve assembly inside the aerosol container and attaching the disc to the can, and for through the valve filling of the foaming agents and the dermatological composition, are as such known in the art.

### Short description of the drawing

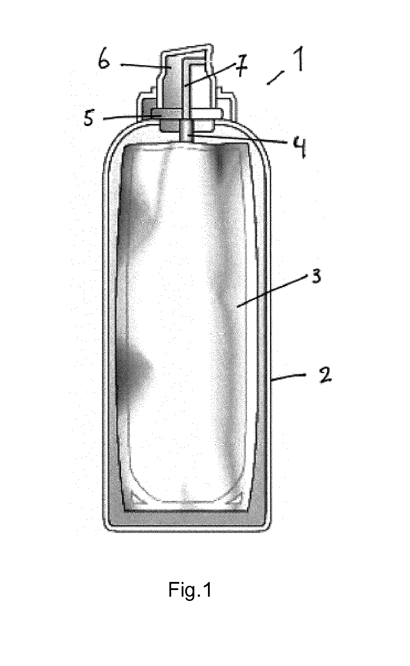

Figure 1 provides a schematic drawing of a bag-on-valve aerosol container.

### Embodiments

The invention is now further illustrated by means of advantageous embodiments of the present invention. Said embodiments are by no means intended to limit the present invention.

A first advantageous embodiment of the present invention is related to a bag-on-valve aerosol container comprising a sunscreen formulation. This embodiment will be referred to as "sunscreen embodiment". The inventors found that when such a sunscreen formulation is applied to the skin as a rich foam, it can easily be spread out without missing any spots. The viscosity of the leave-on dermatological composition used in a formulation according to this embodiment is preferably between 50 and 20,000 mPa.s, more preferably between 100 and 15,000 mPa.s, most preferably between 400 and 13,000 mPa.s.

Preferably, sunscreen formulations according to this embodiment comprise from > 0 to 95 wt.% of one or more oils and active dermatological agents, from > 0 to 15 wt.% of one or more oil-in-water emulsifiers, from > 0 to 10 wt.% of one or more physical foaming agents, > 0 to 30 wt.% of one or more UV-filters, and water.

More preferably, sunscreen formulations according to this embodiment comprise from 1 to 50 wt.% of one or more oils and active dermatological agents, from 0.1 to 10 wt.% of one or more oil-in-water emulsifiers, from 0.5 to 5 wt.% of one or more physical foaming agents, 0.5 to 30 wt.% of one or more UV-filters, and water.

Most preferably, said sunscreen formulations comprise 3.5 to 25 wt.% of the one or more oils and the active ingredients, 3 to 5.5 wt.% of the one or more oil-in-water emulsifiers, 3 to 5 wt.% of the one or more physical foaming agents,10 to 30 wt.% of the one or more UV-filters, and water.

The one or more oils are preferably selected from C₁₂-C₁₅ alkyl benzoates, ethylhexyl cocoate, dicaprylyl carbonate, dibutyl adipate, cyclopentasiloxane and dimethicone.

The one or more emulsifiers are preferably selected from sorbitan stearate, glyceryl stearate, peg-100 stearate, potassium cetyl phosphate, polysorbate 80, bis-peg/ppg-16/16 peg/ppg-16/16 dimethicone, acrylates/vinyl isodecanoate crosspolymer, ceteareth-20, acrylates/C₁₀₋₃₀ alkyl acrylates cross-polymer and laureth-7.

The active ingredients are preferably selected from biosaccharide gum-1, bisabolol, sodium PCA, urea, allantoin, hydrolyzed wheat protein, lactic acid, lysine, PCA, solanum lycopersicum extract, helianthus annuus seed oil, rosmarinus officinalis extract, prunus armeniaca kernel oil, citrus aurantium dulcis extract, tocopheryl acetate and panthenol.

The one or more UV-filters are preferably selected from bis-ethylhexyloxyphenol methoxyphenyl triazine, octocrylene, methylene bis-benzotriazolyl tetramethylbutylphenol, ethylhexyl triazone, butyl methoxydibenzoylmethane, ethylhexyl salicylate, phenylbenzimidazole sulfonic acid and diethylhexyl butamido triazone.

The one or more foaming agents are selected from propane, butane, isobutane and mixtures thereof.

Another preferred embodiment is related to other skin care formulations such as tanning lotions, after-sun lotions and body lotions, wherein the choice of active dermatological ingredients used in such formulations depends on the intended use. Tanning lotions are used to improve tanning of the skin under influence of UV-radiation.

Basically, these skin care formulation fulfil the same criteria and comprise the same ingredients as specified above under the sunscreen embodiment, the most relevant difference being the omission of the UV-filters. It is recognized that omitting one or more UV-filters from a composition might influence the stability of a composition. When needed, the amounts of the ingredients present in a skin care formulation of this embodiment can be adjusted to retain the stability of the formulation. A further stabilizer or further auxiliary agent can even be added to ensure the stability of the formulation. The viscosity of the dermatological composition used in the present skin care formulation is preferably between 900 and 20,000 mPa.s, more preferably between 1,500 and 15,000 mPa.s, most preferably between 2,000 and 10,000 mPa.s.

According to another embodiment of the present invention is related to baby care formulation, wherein the leave-on dermatological composition is a baby care product such as a nappy rash cream comprising at least zinc oxide or tin dioxide as an active dermatological ingredient.

It is generally known that it can be very painful to rub irritated skin with a cream or an ointment. This especially holds for infants, whose skin is more sensitive than the skin of grown-ups. It is a great benefit of the present invention that a caring composition such as a nappy rash cream can now be applied as a foam. The application takes place by pointing the aerosol container to the irritated spots and actuating the aerosol container, without the need for further rubbing.

Preferably, baby care formulations according to this embodiment comprise from 0.1 to 20 wt.% of zinc oxide or titanium dioxide, or a mixture of both, from > 0 to 95 wt.% of one or more oils and active dermatological agents, from > 0 to 15 wt.% of one or more oil-in-water emulsifiers, from > 0 to 10 wt.% of one or more physical foaming agents, and water.

More preferably, baby care formulations according to this embodiment comprise 0.5 to 15 wt.% of zinc oxide or titanium dioxide, or a mixture of both, from 1 to 50 wt.% of one or more oils and further active dermatological agents, from 0.1 to 10 wt.% of one or more oil-in-water emulsifiers, from 0.5 to 5 wt.% of one or more physical foaming agents, and water.

More preferably, said baby care formulations comprise from 0.5 to 5 wt.% of zinc oxide or titanium dioxide, or a mixture of both, from 3.5 to 25 wt.% of the one or more oils and the further active ingredients, 3 to 5.5 wt.% of the one or more oil-in-water emulsifiers, from 3 to 5 wt.% of the one or more physical foaming agents, and water. The baby care formulations optionally comprise from > 0 to 30 wt.% of one or more UV-filters.

The one or more oils can be mineral oils, lipids such as suitable esters, ethers, vegetable oils, fatty acids and phospholipids, or silicone moieties. Said oils are preferably selected from alkyl adipates, caprylic/capric triglyceride, ethylhexyl stearate, dicaprylyl carbonate, dicaprylyl ether, hydrogenated polydecene, pentaerythrityl tetracaprylate/caprate, cyclopentasiloxane, dimethicone.

The one or more emulsifiers are preferably selected from sorbitan stearate, glyceryl stearate, peg-100 stearate, potassium cetyl phosphate, polysorbate 80, bis-peg/ppg-16/16 peg/ppg-16/16 dimethicone, acrylates/vinyl isodecanoate crosspolymer, ceteareth-20, acrylates/C₁₀₋₃₀ alkyl acrylates cross-polymer and laureth-7.

Preferably, the further active ingredients, if any, are selected from biosaccharide gum-1, bisabolol, sodium pca, urea, allantoin, hydrolyzed wheat protein, lactic acid, lysine, pca, solanum lycopersicum extract, helianthus annuus seed oil, rosmarinus officinalis extract, prunus armeniaca kernel oil, citrus aurantium dulcis extract. Advantageously, the one or more foaming agents are selected from propane, butane, isobutane and mixtures thereof.

When a leave-on dermatological composition according to this embodiment is employed, the viscosity preferably is not higher than 20,000 mPa.s, more preferably between 500 and 15,000 mPa.s, most preferably between 1,000 and 10,000 mPa.s.

### Detailed description of the drawing

With reference to figure 1, a bag-on-valve aerosol container 1 according to the invention comprises a bag-on-valve assembly inside an aluminum or tin plate can 2. The bag-on-valve assembly is made up of a product bag 3 attached to a valve 4, which is, via a flange 5, crimp sealed on the can 2. The valve 4 can be both male and female. The can 2 is filled with the pressurizing gas surrounding the product bag 3. This pressurized gas functions to expel a product formulation from the product bag 3, which is a flexible bag. An actuator 6 is connected to the valve 4. When the actuator 6 is depressed, the product formulation is discharged from the bag-on-valve aerosol container 1 through the valve 4 via an outlet channel 7 in the actuator 6. The product formulation is expelled from the product bag 2 due to the force exerted by the compressed air.

### Example 1

The invention is now further explained by means of the following examples, which are not intended to limit the invention in any way.

Leave-on dermatological compositions A-G were prepared by mixing the ingredients as specified in table 1 (in weight per cent).

| table 1: dermatological compositions A-G: ingredients and their amounts (wt.%) | | | | | | | |
|---|---|---|---|---|---|---|---|
| formulation type | Skin Care | Sun Care | Sun Care | Sun Care | Sun Care | Skin Care | Skin Care |
| ingredients | A | B | C | D | E | F | G |
| Water | to 100% | to 100% | to 100% | to 100% | to 100% | to 100% | to 100% |
| Oils | 12.6 | 2 | 9.5 | 3 | 4 | 9 | 10 |
| Waxes | - | 1.5 | - | - | 1 | 3.2 | - |
| Emulsifiers | 1.4 | 1.1 | 3.3 | 6.1 | 5.6 | 1.25 | 3 |
| Humectants | 3 | 3.3 | 3.3 | 3 | 3 | 2.58 | 3 |
| Thickeners | 0.28 | 0.9 | 0.26 | 0.52 | 0.69 | 0.22 | 0.28 |
| UV filters | - | 29.8 | 20.8 | 23.75 | 26.25 | - | - |
| Film formers | - | 1 | 1 | 2 | 2 | - | - |
| Vitamins | 2 | - | - | 0.5 | 0.28 | 0.5 | 1.5 |
| Actives | 0.27 | 3.3 | 3.3 | 0.7 | 0.16 | 0.24 | 3.3 |
| Preservatives | 1,23 | 0,04 | 0,03 | 0,03 | 0,03 | 1,62 | 0,03 |
| pH adjusters | q.s. | q.s. | q.s. | q.s. | q.s. | q.s. | q.s. |
| Perfume | q.s. | q.s. | q.s. | q.s. | q.s. | q.s. | q.s. |

| | | | | | | | |
|---|---|---|---|---|---|---|---|
| q.s. = quantum sufficit | | | | | | | |

The dynamic viscosity of the prepared compositions was measured using a Brookfield RV viscometer. Measured viscosities and relevant measurement conditions are listed in table 2.

Bag-on-valves were brought into aerosol containers and filled by first inserting about 3 wt.% of the one or more physical blowing agents and subsequently filling the compositions A-G into the container. After this, suitable actuators were attached to the aerosol containers.

| table 2: measured viscosity of A-G | | | | | | | |
|---|---|---|---|---|---|---|---|
| | A | B | C | D | E | F | G |
| viscosity (mPa.s) | 2170 | 12050 | 1620 | 444 | 5880 | 4100 | 1140 |
| spindle | 4 | 6 | 2 | 3 | TB | 4 | 4 |
| rotation speed (rpm) | 20 | 20 | 20 | 100 | 20 | 20 | 20 |
| temperature (°C) | 21.2 | 21.6 | 20 | 22.5 | 21.6 | 20.7 | 21.1 |

The dermatological compositions A-G were discharged form bag-on-valve aerosol containers by activating the actuator. All bag-on-valve aerosol containers delivered a rich foam that did not drip from the skin and allowed for easy spreading.

### Example 2

To show that the viscosity of a composition according to the present invnetuion plays a critical role a number of experiments were conducted wherein a skin care formulation with different viscosities and a sun care formulation with different viscosities were compared.

The components of the compositions are shown in table 3.

The viscosities that were measured at 20 °C after 1 minute, are shown in table 4.

| table 3: cosmetic or dermatological compositions H-K ingredient categories and their amounts (wt.%) | | | | |
|---|---|---|---|---|
| | Skin Care formula | Skin Care formula | Sun Care formula | Sun Care formula |
| ingredients | H | I | J | K |
| Water | To 100% | To 100% | To 100% | To 100% |
| Oils | 18 | 18 | 11 | 11 |
| Waxes | 2 | 2 | 2.5 | 1.5 |
| Emulsifiers | 1.1 | 1.1 | 1.2 | 1.2 |
| Humectants | 5 | 5 | 3 | 3 |
| Thickeners | 0.62 | 0.21 | 1 | 0.4 |
| UV filters | | | 17.5 | 17.5 |
| Film formers | | | 1 | 1 |
| Vitamins | 0.52 | 0.52 | | |
| Actives | 3 | 3 | 0.5 | 0.5 |
| Preservatives | 0.85 | 0.85 | 0.15 | 0.15 |
| pH adjusters | q.s. | q.s. | q.s. | q.s. |
| Perfume | q.s. | q.s. | q.s. | q.s. |

| table 4: measured viscosity of compositions H-K | | | | |
|---|---|---|---|---|
| | H | I | J | K |
| viscosity (mPa.s) | 28.800 | 10.080 | 24.350 | 9.420 |
| spindle | TD | TD | 6 | 5 |
| rotation speed (rpm) | 5 | 5 | 20 | 20 |

| | | | | |
|---|---|---|---|---|
| q.s. = quantum sufficit | | | | |

When the compositions were supplied to a bag-on-valve aerosol it appeared that compositions H and J were not so viscous that is was rather difficult for them to be supplied to the aerosol container in an efficient way. Moreover, it appeared that these compositions did not produce a satisfactorily foaming product. In contradistinction therewith, the compositions I and K could easily be filled into the aerosol containers and produced a rich foam that did not drip from the skin and allowed for easy spreading.

## Claims

1. Bag-on-valve aerosol container defining a chamber, wherein a pressurized gas is contained, surrounding a flexible bag, and wherein a closable outlet is in fluid communication with said flexible bag, said flexible bag comprising a leave-on dermatological composition comprising:
- an oil-in-water emulsion, comprising one or more oils, the emulsion having an oil phase to water phase ratio of between 1:1 and 1:25;
- one or more oil-in-water emulsifiers; and
- one or more active dermatological ingredients;
wherein said leave-on dermatological composition has a viscosity at room temperature of between 1 and 20,000 mPa.s and wherein said product compartment further comprises one or more physical foaming agents having a standard boiling point of -50 to 70 °C.

2. Aerosol container according to claim 1, wherein the flexible bag contained in the bag-on-valve aerosol container is a laminated bag comprising at least one metal foil layer and a thermoplastic layer.

3. Aerosol container according to claim 1 or 2, wherein the pressurized gas is nitrogen or air.

4. Aerosol container according to any one of claims 1 to 3, wherein the one or more physical foaming agents are selected from propane, butane, isobutane, pentane, isopentane, dimethyl ether and mixtures thereof.

5. Aerosol container according to any one of claims 1 to 4, wherein the leave-on dermatological composition has a viscosity at room temperature of between 50 and 20,000 mPa.s.

6. Aerosol container according to claim 5, wherein the viscosity at room temperature is between 100 and 15,000 mPa.s.

7. Aerosol container according to any one of claims 1 to 6, wherein the leave-on dermatological composition further comprises one or more auxiliary agents selected from co-emulsifiers, rheological additives, preservatives, pH-adjusting agents, perfumes and aroma's.

8. Aerosol container according to any one of claims 1 to 7, comprising
> 0 to 95 wt.% of the one or more oils and active dermatological agents;
> 0 to 15 wt.% of the one or more oil-in-water emulsifiers; and
> 0 to 10 wt.% of the one or more physical foaming agents,
wherein the one or more oils are preferably selected from C12-15 alkyl benzoates, ethylhexyl cocoate, dicaprylyl carbonate, dibutyl adipate, cyclopentasiloxane and dimethicone,
wherein the one or more oil-in-water emulsifiers are preferably selected from sorbitan stearate, glyceryl stearate, peg-100 stearate, potassium cetyl phosphate, polysorbate 80, bis-peg/ppg-16/16 peg/ppg-16/16 dimethicone, acrylates/vinyl isodecanoate crosspolymer, ceteareth-20, acrylates/C₁₀₋₃₀ alkyl acrylates cross-polymer and laureth-7,
wherein the one or more active ingredients are preferably selected from biosaccharide gum-1, bisabolol, sodium PCA, urea, allantoin, hydrolyzed wheat protein, lactic acid, lysine, PCA, solanum lycopersicum extract, helianthus annuus seed oil, rosmarinus officinalis extract, prunus armeniaca kernel oil, citrus aurantium dulcis extract, tocopheryl acetate and panthenol, and
wherein the one or more foaming agents are preferably selected from propane, butane, isobutane, or mixtures thereof.

9. Aerosol container according to claim 8, wherein the leave-on dermatological composition is a baby care composition comprising zinc oxide and/or titanium dioxide as an active ingredient.

10. Aerosol container according to any one of claims 1 to 9, wherein the leave-on dermatological composition is a formulation further comprising one or more UV-filters.

11. Aerosol container according to claim 10, wherein the one or more UV-filters are selected from bis-ethylhexyloxyphenol methoxyphenyl triazine, octocrylene, methylene bis-benzotriazolyl tetramethylbutylphenol, ethylhexyl triazone, butyl methoxydibenzoylmethane, ethylhexyl salicylate, phenylbenzimidazole sulfonic acid and diethylhexyl butamido triazone.

12. Aerosol container according to claim 10 or 11, comprising
> 0 to 95 wt.% of the one or more oils and active dermatological agents;
> 0 to 15 wt.% of the one or more oil-in-water emulsifiers; and
> 0 to 10 wt.% of the one or more physical foaming agents,
and
> 0 to 30 wt.% of the one or more UV-filters;
wherein the one or more oils are preferably selected from C₁₂₋₁₅ alkyl benzoates, ethylhexyl cocoate, dicaprylyl carbonate, dibutyl adipate, cyclopentasiloxane and dimethicone,
wherein the one or more oil-in-water emulsifiers are preferably selected from sorbitan stearate, glyceryl stearate, peg-100 stearate, potassium cetyl phosphate, polysorbate 80, bis-peg/ppg-16/16 peg/ppg-16/16 dimethicone, acrylates/vinyl isodecanoate crosspolymer, ceteareth-20, acrylates/C₁₀₋₃₀ alkyl acrylates cross-polymer and laureth-7,
wherein the one or more active ingredients are preferably selected from biosaccharide gum-1, bisabolol, sodium PCA, urea, allantoin, hydrolyzed wheat protein, lactic acid, lysine, PCA, solanum lycopersicum extract, helianthus annuus seed oil, rosmarinus officinalis extract, prunus armeniaca kernel oil, citrus aurantium dulcis extract, tocopheryl acetate and panthenol, and
wherein the one or more foaming agents are selected from propane, butane, isobutane and mixtures thereof.

13. Method for filling a bag-on-valve aerosol container, comprising the steps of:
(a) pressurizing a can (2) with the pressurizing gas;
(b) bringing a valve assembly comprising a flexible bag (3), a valve (4) and a disc (5) into the can such that the pressurizing gas surrounds the flexible bag;
(c) attaching the valve assembly with its disc (5) to the can (2) such that the aerosol container is closed; and
(d) filling through the valve the one or more physical foaming agents as liquids into the flexible inner bag and subsequently adding thereto the leave-on dermatological composition.
